# EUROPÄISCHE PATENTSCHRIFT

(11) **EP 0 579 996 B1**
(45) Veröffentlichungstag und Bekanntmachung des Hinweises auf die Patenterteilung: **03.05.1995**
(21) Anmeldenummer: 93110567.0
(22) Anmeldetag: 02.07.1993
(51) Int. Cl.: A61K 47/40, A61K 31/495

(54) **5,10-Methylentetrahydrofolsäure-Cyclodextrin-Einschlussverbindungen**
Inclusion compounds of 5,10-methylenetetrahydrofolic acid and cyclodextrin
Composés d'inclusion de l'acide méthylène-5,10-tétrahydrofolique et de cyclodextrine

(30) Priorität: 13.07.1992 CH 2192/92
(43) Veröffentlichungstag der Anmeldung: 26.01.1994
(73) Patentinhaber: EPROVA Aktiengesellschaft, CH-8200 Schaffhausen (CH)
(72) Erfinder: Müller, Hans Rudolf, CH-8207 Schaffhausen (CH); Conti, Josef, CH-8203 Schaffhausen (CH); Ulmann, Martin, CH-8447 Dachsen (CH)

(56) Entgegenhaltungen:
- EP-A- 401 895
- EP-A- 0 427 078
- WO-A-91/17660
- Science, Band 232, 1986, Seiten 1132-5
- L.J. Machlin, Handbook of Vitamins, M.Decker Inc. N.Y./Basel, T.Brody, Seite 457
- J.Am.Chem.Soc., Band 82, 1960, Seite 4921
- J.Biol.Chem., Band 241, 1966, Seite 5851
- Proc.Natl.Acad.Sci.USA, Band 76, 1979, Seiten 1456-1460
- Folates and Pterins, R.L. Blakley et al., John Wiley & Sons, New York, Band 1, Seiten98-99
- J.Food Science, Band 44, 1979, Seiten 717-722

## Beschreibung

Die Erfindung betrifft α-, β-, γ-, Hydroxypropyl-β-, Hydroxypropyl-γ-, Dimethyl-β- und Dimethyl-γ-Cyclodextrin Einschlussverbindungen der (6R)-, (6S)- und (6R,S)-5,10-Methylentetrahydrofolsäure. Die überraschend gute Stabilität dieser Verbindungen ermöglicht erstmals deren pharmazeutische Verwendung.

Die Erfindung betrifft ausserdem ein Verfahren zur Herstellung der genannten Substanzen sowie auch deren Verwendung zur Herstellung von Arzneimitteln.

Im folgenden ist unter Hydroxypropyl-Cyclodextrin jeweils Hydroxypropyl-Cyclodextrin (0.6), Hydroxypropyl-Cyclodextrin (0.9), 3-Hydroxypropyl-Cyclodextrin, 2,3-Dihydroxypropyl-Cyclodextrin und vor allem 2-Hydroxypropy-Cyclodextrin zu verstehen. Ebenfalls sind im folgenden unter 5,10-Methylentetrahydrofolsäure auch 5,10-Methylentetrahydrofolsäure-Salze, vor allem Alkali- und Erdalkalisalze zu verstehen.

Tetrahydrofolate sind die biologisch aktiven Formen der Folsäure (Folsäure-Co-Faktoren). Als Arzneimittel werden Tetrahydrofolate vorwiegend als Calciumsalz der 5-Formyl-5,6,7,8-tetrahydrofolsäure [Leucovorin] verwendet, z.B. zur Verstärkung des therapeutischen Effektes von 5-Fluoruracil oder z.B. als Rescue-Substanz beim Einsatz von Methotrexat in der Krebstherapie.
Im Organismus wird (6S)-5-Formyltetrahydrofolsäure in (6R)-5,10-Methylentetrahydrofolsäure umgewandelt, welche als Co-Faktor mit aus 5-Fluoruracil (5-FU) gebildetem 5-Fluor-Desoxyuridinmonophosphat (5-F-dUMP) und Thymidylat-Synthetase (TS) einen cytostatisch wirksamen kovalenten temären Komplex: 5-F-dUMP/TS/5,10-Methylentetrahydrofolsäure bildet. Siehe dazu W.A. Bleyer, Cancer March 15 Supplement 1989: S. 995-1007 sowie E.L.R. Stokstad, Folic Acid Metabolism in Health and Disease 1990 (Wiley-Liss Inc), Seite 9.
Es wäre daher von Vorteil, anstelle von Leucovorin (5-Formyltetrahydrofolsäure) direkt den Co-Faktor 5,10-Methylentetrahydrofolsäure zu verwenden. Siehe dazu WO 91/17660 Seite 5, Zeilen 24 bis 35. Bisher scheiterte dieses Unterfangen an der nicht ausreichenden Reinheit und Stabilität von 5,10-Methylentetrahydrofolsäure und deren Salzen. Vergleiche dazu EP 0 409 125, die darin beschriebenen Gehalte an (6R,S)-5,10-Methylentetrahydrofolsäure liegen im allgemeinen zwischen 85 und 90%. Einzig vom Calcium- und Magnesiumsalz der (6R,S)-5,10-Methylentetrahydrofolsäure wurden Präparate mit einem Gehalt an 96.5-98.8% erhalten. Die Stabilität der Salzlosungen ist jedoch sehr kritisch. Bei pH 9 sind nach 6 Stunden nur noch etwa 85% der ursprünglichen Menge 5,10-Methylentetrahydrofolsäure vorhanden. Siehe dazu EP 0 409 125, Tabelle, Seite 14.

5,10-Methylentetrahydrofolsäure steht in Lösung im Gleichgewicht mit Formaldehyd und Tetrahydrofolsäure. Siehe dazu L.J. Machlin, Handbook of Vitamins, 2nd Ed (Marcel Decker Inc., N.Y./Basel), T. Brody Seite 457; M.J. Osborn, et al, J. Am. Chem. Soc. 82, 4921 (1960), R.G. Kallen, et al., J. Biol. Chem., 241, 5851 (1966), Moran et al, Proc. Natl. Acad. Sci. USA 76, 1456-1460 (1979). Dieses Gleichgewicht steht der parenteralen Anwendung von 5,10-Methylentetrahydrofolsäure entgegen. Auch in WO 91/17660, die sich intensiv mit den Vorteilen der Verwendung von 5,10-Methylentetrahydrofolsäure gegenüber andern Tetrahydrofolsäure-Derivaten auseinandersetzt, wird keine Möglichkeit zur Stabilisierung von 5,10-Methylentetrahydrofolsäure aufgezeigt. Diese Stabilisierung ist jedoch die Grundlage, die eine pharmazeutische Verwendung von 5,10-Methylentetrahydrofolsäure erst ermöglicht. Bisher bekannte Technologien, wie die zur Stabilisierung von 5-Formyltetrahydrofolsäure verwendete Zugabe von Ascorbinsäure, Parabenen, Merkaptoalkoholen oder Trometamol, siehe dazu EP 0 416 232, oder die Stabilisierung des Calciumsalzes von 5-Formyltetrahydrofolsäure durch Zusatz eines Komplexierungsmittels für das Erdalkaliion, siehe dazu EP 0 401 895, können nicht auf 5,10-Mehylentetrahydrofolsäure angewendet werden oder zeigen nur beschränkten Erfolg, z.B. durch Verlangsamung des oxidativen Abbaus der Tetrahydrofolsäure und Tetrahydrofolsäure-Derivate. Keine dieser Methoden beeinflusst jedoch das Gleichgewicht in dem 5,10-Methylentetrahydrofolsäure zu Tetrahydrofolsäure und freiem Formaldehyd steht.

Ebenfalls klar nur auf die Hemmung des oxidativen Abbaus ausgerichtet ist der Einsatz von β-Cyclodextrin zur Stabilisierung von Dihydrofolsäure, die als Substrat in der biochemischen Analytik von Methotrexat gebraucht werden kann (JP 58-48933 Sho) und der Einsatz von Cyclodextrin zur Stabilisierung von Leucovorin (EP 0 427 078).

Es wurde nun überraschend gefunden, dass Tetrahydrofolsäure auch in Gegenwart von Cyclodextrinen die Kondensationsreaktion mit Formaldehyd eingeht und vor allem, dass sich nach der Reaktion in wässriger Lösung stabile Einschlussverbindungen von 5,10-Methylentetrahydrofolsäure im entsprechenden Cyclodextrin ergeben und dadurch erstmals die therapeutische Anwendung von 5,10-Methylentetrahydrofolsäure in wässrigen Lösungen ermöglicht wird.

Die Stabilitätserhöhung ist umso mehr überraschend, als dass bisherige Messungen die Schlussfolgerung zuliessen, es trete praktisch keine Wechselwirkung von Tetrahydrofolaten mit Cyclodextrin ein. Siehe dazu D.W. Armstrong, et al, Science, 232, 1132-5, (1986), im speziellen Seite 134, Spalte 2, Zeile 2. Durch eigene Messungen konnte bestätigt werden, dass Cyclodextrin mit 5-Formyltetrahydrofolsäure nicht und mit 5-Methyltetrahydrofolsäure nur äusserst schwach in Wechselwirkung tritt. Eine Stabilisierung von 5,10-Methylentetrahydrofolsäure in bezug auf den chemischen Abbau, aber vor allem auch in bezug auf eine Verschiebung des Gleichgewichts von freiem Formaldehyd und Tetrahydrofolsäure hin zu 5,10-Methylentetrahydrofolsäure war somit nicht zu erwarten.

Die Cyclodextrin-Einschlussverbindungen werden bevorzugt aus dem durch die Europäische Patentanmeldung EP-0 495 204 leicht zugänglich gewordenen (6S)-, (6R)- und (6R,S)-Tetrahydrofolsäuresulfat oder Sulfonsäuresalz in situ oder nach vorangegangener Isolierung oder Freisetzung und Aufreinigung durch Umsetzung mit Formaldehyd in Gegenwart vom entsprechenden Cyclodextrin hergestellt. Zur quantitativen Umsetzung der Tetrahydrofolsäure sind dabei nur Weine molare Überschüsse an Formaldehyd von max. 10-20% notwendig. Die Reaktion erfolgt dabei bevorzugt im pH-Bereich zwischen 8 und 9. Die Einschlussverbindung kann jedoch auch durch Eintragen von (6R)-, (6S)- oder (6R,S)-5,10-Methylentetrahydrofolsäure in eine Cyclodextrin-Lösung oder Cyclodextrin-Aufschlämmung oder durch Verreiben von (6R)-, (6S)- oder (6R,S)-5,10-Methylentetrahydrofolsäure und Cyclodextrin in fester Form erfolgen. Die erhaltenen Produkte sind in Lösung bei Raumtemperatur stabil. Sie sind geeignet als Bestandteile von parenteralen Arzneimittelformen oder als Ausgangsmaterial zur Herstellung von oralen Arzneimittelformen. Sowohl die oralen als auch die parenteralen Arzneimittelformen sind z.B. zur Krebstherapie, zur Behandlung von bestimmten Anämie Formen, von Autoimmunkrankheiten und von neuralen Störungen geeignet.

Gegenstand der Erfindung sind neue α-, β-, γ-, Hydroxypropyl-β-, Hydroxypropyl-γ-, Dimethyl-β- und Dimethyl-γ-Cyclodextrin-Einschlussverbindungen der (6R)-, (6S)-und (6R,S)-5,10-Methylentetrehydrofolsäure. Diese Produkte sind in wässriger Lösung stabil. Sie eignen sich auch zur Herstellung von parenteralen Arzneimittelformen, z.B. in der Krebstherapie.

Aufgrund der hohen Löslichkeit und physiologisch guten Verträglichkeit eignen sich vor allem Alkali- oder Erdalkalisalze, im speziellen das Natrium-, Kalium-, Calcium- oder Magnesiumsalz der 5,10-Methylentetrahydrofolsäure zur Herstellung dieser Verbindungen. Das Magnesiumsalz der 5,10-Methylentetrahydrofolsäure weist bei ähnlicher Reinheit gegenüber dem Calciumsalz eine ca zehnfach höhere, das Natrium- und das Kaliumsalz eine nochmals höhere Löslichkeit auf. Diese Substanzen sind daher bei der Herstellung pharmazeutischer Produkte speziell hervorzuheben.

Bevorzugte Verbindungen sind:
Alkali- oder Erdalkalisalze der (6R)- und (6S)-5,10-Methylentetrahydrofolsäure mit β-Cyclodextrin
Alkali- oder Erdalkalisalze der (6R)- und (6S)-5,10-Methylenteträhydrofolsäure mit Hydroxypropyl-β-Cyclodextrin
Alkali- oder Erdalkalisalze der (6R)- und (6S)-5, 10-Methylentetrahydrofolsäure mit Hydroxpropyl-γ-Cyclodextrin
Alkali- oder Erdalkalisalze der (6R)- und (6S)-5,10-Methylentetrahydrofolsäure mit γ-Cyclodextrin
Alkali- oder Erdalkalisalze der (6R)- und (6S)-5,10-Methylentetrahydrofolsäure mit Dimethyl-β-Cyclodextrin
Wobei jeweils die natürliche (6R)-Form der 5,10-Methylentetrahydrofolsäure zur Herstellung der Einschlussverbindung zu bevorzugen ist.

Ein weiterer Gegenstand der Erfindung ist das Verfahren zur Herstellung von stabilen Cyclodextrin-Einschlussverbindungen der 5,10-Methylentetrahydrofolsäure, welches dadurch gekennzeichnet ist, dass man 5,10-Methylentetrahydrofolsäure oder ein Derivat der 5,10-Methylentetrahydrofolsäure mit α-, β- oder γ-Cyclodextrin oder einem Derivat von α-, β- oder γ-Cyclodextrin umsetzt oder vorzugsweise Tetrahydrofolsäure oder ein Salz der Tetrahydrofolsäure mit dem entsprechenden Cyclodextrin umsetzt, falls erwünscht umsalzt oder freisetzt, und danach in situ durch Zugabe von Formaldehyd die 5,10-Methylentetrahydrofolsäure-Cyclodextrin-Einschlussverbindungen herstellt und falls erwünscht die erhaltene 5,10-Methylentetrahydrofolsäure-Cyclodextrin-Einschlussverbindung isoliert.

Bevorzugte Salzpartner der 5,10-Methylentetrahydrofolsäure bzw. der Tetrahydrofolsäure sind dabei pharmazeutisch verträgliche Kationen wie Natrium, Kalium, Magnesium, Calcium oder Anionen wie Sulfate, Sulfonate oder Halogenide.

Bevorzugte Cyclodextrin-Einschlussverbindungen entstehen mit Cyclodextrinen wie β-, Hydroxypropyl-β-, Dimethyl-β-, γ-, Hydroxypropyl-γ- oder Dimethyl-γ-Cyclodextrin. Die Cyclodextrine werden bevorzugt in mindestens molaren Verhältnissen zur 5,10-Methylentetrahydrofolsäure von 1:1 oder 2:1 eingesetzt. Der Einsatz hochkonzentrierter Cyclodextrin-Lösungen ist dabei von Vorteil. Es können dabei auch Mischungen von verschiedenen Cyclodextrinen eingesetzt werden. Je nach Verwendungszweck kann dabei ein bestimmtes Salz der 5,10-Methylentetrahydrofolsäure, ein bestimmtes Cyclodextrin oder ein bestimmtes Verhältnis von 5,10-Methylentetrahydrofolsäure zum Cyclodextrin bevorzugt werden. Die optimalen Bedingungen können durch einfache Versuche bestimmt werden.

Die Umsetzung von Tetrahydrofolsäure mit Formaldehyd in Gegenwart von Cyclodextrin erfolgt vorzugsweise in einem Lösungsmittel bestehend aus Wasser oder einem mit Wasser mischbaren organischen Lösungsmittel wie einer niedrigen aliphatischen Carbonsäure oder einem niedrigen Alkohol.

Aufgrund der Oxidationsempfindlichkeit der Tetrahydrofolsäure empfiehlt sich die Verwendung eines Oxidationsschutzes.

Die Isolierung der Cyclodextrin-Einschlussverbindung erfolgt mittels bekannter Techniken wie z.B. Abdampfen des Lösungsmittels bei erhöhter Temperatur im Vakuum, Kristallisation, Lyophilisierung oder Fällung durch Zugabe eines organischen Lösungsmittels.
Die Herstellung der Cyclodextrin-Einschlussverbindung kann auch durch Verreiben von 5,10-Methylentetrehydrofolsäure und Cyclodextrin in fester Form erfolgen.

Die Erfindung betrifft auch die Verwendung der (6R)-, (6S)- oder (6R,S)-5,10-Methylentetrahydrofolsäure oder deren Salzen als Einschlussverbindung in α-, β- oder γ-Cyclodextrin oder deren Derivaten als Bestandteil oder als Ausgangsmaterial zur Herstellung von Arzneimitteln.

### Beispiele zur Illustrierung der Erfindung

### Beispiel 1

### Stabilität von Cyclodextrin-Einschlussverbindungen der 5,10-Methylentetrahydrofolsäure und deren Salzen in Lösung

### a) Stabilität der Natrium- und Calciumsalz-Lösungen von (6R)- und (6S)-5,10-Methylentetrahydrofolsäure bei 23 C° in Phosphatpuffer

| | *Wochen* | | | | | |
|---|---|---|---|---|---|---|
| | *1* | *2* | *4* | *5* | *7* | *14* |
| Na-Salz (6R)-CH₂-THF ohne Cyclodextrin unter Stickstoff | 91.1 | 64.4 | 30.5 | 18.1 | 16.7 | - |
| Na-Salz (6R)-CH₂-THF β-Cyclodextrin | - | 92.3 | - | - | - | 88.5 |
| Na-Salz (6S)-CH₂-THF β-Cyclodextrin | 100.0 | 84.8 | - | 75.0 | - | - |
| Na-Salz (6R)-CH₂-THF α- + β-Cyclodextrin (1:1:1) | 100.0 | 95.8 | 94.3 | 83.2 | 78.5 | 73.0 |
| Ca-Salz (6R)-CH₂-THF α- + β-Cyclodextrin (1:1:1) | - | 80.2 | 57.3 | 50.2 | 47.4 | 44.0 |
| CH₂-THF = 5,10-Methylentetrahydrofolsäure | | | | | | |

### b) Stabilität der Natriumsalz-Lösungen von (6R)-5,10-Methylentetrahydrofolsäure bei 60°C/Stresstest in Phosphatpuffer

| | *Stunden* | | | | | |
|---|---|---|---|---|---|---|
| | *3* | *6* | *18* | *24* | *30* | *48* |
| ohne Cyclodextrin | 77.7 | 34.4 | 2.8 | - | 0.8 | - |
| α-Cyclodextrin | - | 84.1 | - | - | - | 12.6 |
| β-Cyclodextrin | 91.2 | - | - | 75.2 | - | - |
| Dimethyl-β-Cyclodextrin | 88.9 | - | - | 31.5 | - | - |
| Hydroxypropyl-β-Cyclodextrin | 90.4 | - | - | 70.5 | - | - |
| γ-Cyclodextrin | 95.4 | - | - | 63.5 | - | - |
| Hydroxypropyl-γ-Cyclodextrin | 97.4 | - | - | 73.7 | - | - |

### c) Stabilität der Calciumsalz-Lösung von (6R)-5,10-Methylentetrahydrofolsäure bei 60°C/Stresstest in Phosphatpuffer (PBS)

| | *Stunden* | | | | | |
|---|---|---|---|---|---|---|
| | *3* | *6* | *18* | *24* | *30* | *48* |
| ohne Cyclodextrin | 74.4 | 53.8 | - | 7.7 | 2.6 | 0.1 |
| α-Cyclodextrin | 74.5 | 56.4 | - | 7.8 | 3.8 | 0.8 |
| Hydroxypropyl-α-Cyclodextrin (0.6) | 75.3 | 57.1 | - | 7.8 | 2.7 | 0.7 |
| β-Cyclodextrin | 87.7 | 77.8 | - | 42.0 | 33.3 | 14.8 |
| Dimethyl-β-Cyclodextrin (1.8) | 80.2 | 69.1 | - | 29.6 | 22.2 | 7.4 |
| Hydroxypropyl-β-Cyclodextrin (0.6) | 83.8 | 71.2 | - | 28.8 | 22.5 | 6.2 |
| Hydroxypropyl-β-Cyclodextrin (0.9) | 83.5 | 69.6 | - | 29.1 | 21.5 | 7.6 |
| γ-Cyclodextrin | 81.2 | 66.2 | - | 16.2 | 8.8 | 1.2 |
| Hydroxypropyl-γ-Cyclodextrin (0.6) | 95.1 | 81.4 | - | 13.6 | 6.8 | 1.0 |

### d) Stabilität der Magnesiumsalz-Lösung von (6R)-5,10-Methylentetrahydrofolsãure bei 60°C/Stresstest in Phosphatpuffer (PBS)

| | *Stunden* | | | | |
|---|---|---|---|---|---|
| | *3* | *6* | *18* | *24* | *30* |
| ohne Cyclodextrin | 72.7 | 53.4 | - | 7.6 | 2.4 |
| α-Cyclodextrin | 76.5 | 58.8 | - | 12.9 | 4.9 |
| Hydroxypropyl-α-Cyclodextrin (0.6) | 80.2 | 67.4 | - | 14.0 | 6.3 |
| β-Cyclodextrin | 79.1 | 61.6 | - | 26.7 | 17.4 |
| Dimethyl-β-Cyclodextrin (1.8) | 73.6 | 56.3 | - | 19.5 | 11.5 |
| Hydroxypropyl-β-Cyclodextrin (0.6) | 83.3 | 64.3 | - | 25.0 | 14.3 |
| Hydroxypropyl-β-Cyclodextrin (0.9) | 82.6 | 65.1 | - | 24.4 | 12.8 |
| γ-Cyclodextrin | 82.1 | 64.3 | - | 20.2 | 10.8 |
| Hydroxypropyl-γ-Cyclodextrin (0.6) | 81.2 | 64.7 | - | 17.6 | 8.9 |

Die in den Tabellen angegebenen Werte entsprechen jeweils dem Gehalt an 5,10-Methylentetrehydrofolsäure in Prozent des Startwertes (t = 0).

Zur Stabilitätsbestimmung eingesetzt wurden Lösungen mit einer Konzentration von ca 4% 5,10-Methylentetrahydrofolsäure bei pH 7, hergestellt nach den Beispielen 3-20. Ziel der Versuche war es, jeweils innerhalb einer der Tabellen a, b, c oder d vergleichbare Messwerte zu erhalten. Unter optimal gewählten Bedingungen, die leicht in einfachen Versuchen durch Variation der Konzentration von 5,10-Methylentetrahydrofolsäure resp. Cyclodextrin bzw. durch Variation von deren Konzentrationsverhältnis oder durch die Wahl des Salzes der 5,10-Methylentetrahydrofolsäure bzw. durch die Wahl des Lösungsmittels bestimmt werden können, liegt die Stabilität der Einschlussverbindung deutlich höher.

Zur Herstellung der Substanzen in den folgenden Beispielen wurden Cyclodextrine mit folgendem Wassergehalt eingesetzt: α-Cyclodextrin 11.5%, β-Cyclodextrin 15.6%, Dimethyl-β-Cyclodextrin 12.7%, Hydroxypropyl-β-Cyclodextrin 6.4%, γ-Cyclodextrin 9.8%, Dimethy-γ-Cyclodextrin 9.2%, Hydroxypropyl-γ-Cyclodextrin 6.0% (alle Werte bestimmt mittels Thermogravimetrie).

### Beispiel 2

### Chemical-Shift von Cyclodextrin-Einschlussverbindungen der 5,10-Methylentetrahydrofolsäure im Vergleich zum Gemisch von Cyclodextrin und 5-Formyltetrahydrofolsäure

In wässrigen Lösungen wurde die Änderung der Abschirmung (Δppm) der ¹³C-Signale von 5,10-Methylentetrahydrofolsäure bzw. der ¹³C-Signale von 5-Formyltetrahydrofolsäure und der ¹H-Signale von 5-10-Methylentetrahydrofolsäure durch die Bildung der Einschlussverbindung mit β-Cyclodextrin bei pH 9 gemessen.

| **¹³C-Spektren** | | **Δppm** |
|---|---|---|
| 5,10-Methylentetrahydrofolsäure | C-7 | + 0.532 |
| | C-9 | + 0.908 |
| | C-11 | + 1.301 |

| **¹³C-Spektren** | | **Δppm** |
|---|---|---|
| 5-Formyltetrahydrofolsäure | C-7 | + 0.013 |
| | C-9 | + 0.013 |
| | C-11 | - 0.007 |

| **¹H-Spektren** | | **Δppm** |
|---|---|---|
| 5,10-Methylentetrahydrofolsäure | H₂-7 | + 0.20 |
| | H-3'/H-5' | - 0.15 |
| | H-2'/H-6' | + 0.07 |
| | H₂-γ-Glu | - 0.08 |

Aus den Δppm-Werten der ¹³C-Spektren der 5-Formyltetrahydrofolsäure ist deutlich ersichtlich, dass Cyclodextrin mit dieser Substanz nicht in Wechselwirkung steht. Im Gegensatz dazu weisen die Δppm-Werte der ¹³C-Spektren und der ¹H-Spektren der 5,10-Methylentetrahydrofolsäure auf eine ausgesprochen starke Wechselwirkung, speziell im Pteridinteil des Moleküls hin.

### Beispiel 3

### β-Cyclodextrin-Einschlussverbindungen der (6R)-5,10-Methylentetrahydrofolsäure

22.7 g β-Cyclodextrin und 4.88 g (6S)-Tetrahydrofolsäure werden unter Stickstoff bei Raumtemperatur in 2'000 ml Wasser suspendiert. Danach werden 1.3 ml Formalinlösung (36.2%) zugetropft Durch leichtes Erwärmen der Suspension auf ca 40°C erhält man eine praktisch Ware Lösung, die nach einer Klarfiltration am Rotationsverdampfer bei 20° C/1 mbar zur Trockene eingeengt wird.

Man erhält 25.8 g β-Cyclodextrin-Einschlussverbindung der (6R)-5,10-Methylentetrehydrofolsäure mit einem Gehalt an (6R)-5,10-Methylentetrahydrofolsäure von 13.3% (bestimmt mittels HPLC).

### Beispiel 4

### β-Cyclodextrin-Einschlussverbindung des Natriumsalzes der (6R)-5,10-Methylentetrahydrofolsäure

a) 22.7 g β-Cyclodextrin werden in 650 ml Wasser suspendiert. Bei Raumtemperatur werden unter Stickstoff 4.8 g (6S)-Tetrahydrofolsäure zugegeben. Dabei wird der pH der Suspension bei 7-9 gehalten (16.3 ml NaOH 1n). Danach werden 0.93 ml Formalinlösung (36.2%) zugetropft. Die entstandene Lösung wird klarfiltriert und nach ca 20 Minuten Nachreaktionszeit bei Raumtemperatur bei 20° C/1 mbar zur Trockene eingeengt.
   Man erhält 26.3 g β-Cyclodextrin-Einschlussverbindung des Natriumsalzes der (6R)-5,10-Methylentetrehydrofolsäure mit einem Gehalt an (6R)-5,10-Methylentetrahydrofolsäure von 17.9% (bestimmt mittels HPLC).
b) Ein ähnliches Produkt, wie unter Beispiel 4 a) beschrieben, erhält man durch intensives Verreiben von 10 g feuchtem β-Cyclodextrin und 2.6 g (6R)-5,10-Methylentetrahydrofolsäure Natriumsalz.
   Stabilität Feststoff: Gehalt der (6R)-5,10-Methylentetrahydrofolsäure nach 113 Tg/- 25° C 100.0%, nach 63 Tg/+ 23° C 98.2%.

### Beispiel 5

### β-Cyclodextrin-Einschlussverbindung des Calciumsalzes der (6R)-5,10-Methylentetrahydrofolsäure

5.78 g β-Cyclodextrin und 320 mg Calciumhydroxid werden in 30 ml Wasser aufgeschlämmt. Unter Stickstoff werden 1.97 g (6S)-Tetrahydrofolsäure zugegeben. Danach werden 28 ml Wasser und 0.37 ml Formalinlösung (36.2%) zugegeben. Die entstandene Lösung wird klarfiltriert und die Einschlussverbindung mit 300 ml Ethanol bei 0°C aus dem Filtrat ausgefällt. Das Produkt wird mit Ethanol/Wasser gewaschen und bei 20° C getrocknet.

Man erhält 7.7 g β-Cyclodextrin-Einschlussverbindung des Calciumsalzes der (6R)-5,10-Methylentetrahydrofolsäure mit einem Gehalt an (6R)-5,10-Methylentetrahydrofolsäure von 25.6% (bestimmt mittels HPLC).

### Beispiel 6

### β-Cyclodextrin-Einschlussverbindung des Kaliumsalzes der (6R)-5,10-Methylentetrahydrofolsäure

Die β-Cyclodextrin-Einschlussverbindung des Kaliumsalzes der (6R)-5,10-Methylentetrahydrofolsäure wird in analoger Weise hergestellt wie im Beispiel 4 beschrieben.

### Beispiel 7

### β-Cyclodextrin-Einschlussverbindung des Magnesiumsalzes der (6R)-5,10-Methylentetrahydrofolsäure

Die β-Cyclodextrin-Einschlussverbindung des Magnesiumsalzes der (6R)-5,10-Methylentetrahydrofolsäure wird in analoger Weise hergestellt wie im Beispiel 5 beschrieben.

### Beispiel 8

### α- und β-Cyclodextrin-Einschlussverbindung des Calciumsalzes der (6R)-5,10-Methylentetrahydrofolsäure

10.85 g α-Cyclodextrin, 12.9 g β-Cyclodextrin und 744 mg Calciumhydroxid werden in 100 ml Wasser aufgeschlämmt. Unter Stickstoff werden 4.93 g (6S)-Tetrahydrofolsäure, weitere 31 mg Calciumhydroxid und 0.92 ml Formalinlösung (36.2%) zugegeben. Nach Ablauf einer Nachreaktionszeit von ca 20 Minuten wird die Lösung klarfiltriert und aus dem Filtrat durch Zusatz von 750 ml Ethanol bei 0°C die Einschlussverbindung ausgefällt, mit Ethanol/Wasser gewaschen und bei 20° C getrocknet.

Man erhält 29.2 g α-/β-Cyclodextrin-Einschlussverbindung der (6R)-5,10-Methylentetrahydrofolsäure mit einem Gehalt an (6R)-5,10-Methylentetrahydrofolsäure von 16.5% (bestimmt mittels HPLC).

### Beispiel 9

### β-Cyclodextrin-Einschlussverbindung des Natriumsalzes der (6S)-5,10-Methylentetrahydrofolsäure

2.27 g β-Cyclodextrin werden in 100 ml Wasser suspendiert Bei Raumtemperatur werden unter Stickstoff 608 mg (6S)-5,10-Methylentetrahydrofolsäure zugegeben.

Dabei wird der pH der Suspension bei 7-9 gehalten (2.7 ml NaOH 1n). Die entstandene Lösung wird klarfiltriert und lyophilisiert.

Man erhält 2.75 g β-Cyclodextrin-Einschlussverbindung des Natriumsalzes der (6S)-5,10-Methylentetrahydrofolsäure mit einem Gehalt an (6S)-5,10-Methylentetrahydrofolsäure von 16.0% (bestimmt mittels HPLC).

Stabilität Feststoff: Gehalt an (6S)-5,10-Methylentetrahydrofolsäure-THF nach 156 Tg/- 25° C 100.0%.

### Beispiel 10

### β-Cyclodextrin-Einschlussverbindung des Natriumsalzes der (6R)-5,10-Methylentetrahydrofolsäure aus dem Sulfat

22.7 g β-Cyclodextrin werden in 1130 ml Wasser suspendiert. Bei Raumtemperatur werden unter Stickstoff 5.4 g (6R)-5,10-Methylentetrahydrofolsäure-Sulfat zugegeben. Dabei wird der pH der Suspension bei 7-9 gehalten (33 ml NaOH 1n). Die entstandene Lösung wird klarfiltriert und lyophilisiert.

Man erhält 28.4 g β-Cyclodextrin-Einschlussverbindung des Natriumsalzes der (6R)-5,10-Methylentetrahydrofolsäure mit einem Gehalt an (6R)-5,10-Methylentetrahydrofolsäure von 15.3% (bestimmt mittels HPLC).

Stabilität Feststoff: Gehalt an Natriumsalz der (6R)-5,10-Methylentetrahydrofolsäure nach 154 Tg/- 25° C 99.5%.

### Beispiel 11

### β-Cyclodextrin-Einschlussverbindung der (6R)-5,10 Methylentetrahydrofolsäure aus einer Lösung

2.27g β-Cyclodextrin werden in 150 ml Wasser bei 25°C gelöst. Unter Stickstoff werden 608 mg (6R)-5,10-Methylentetrahydrofolsäure zugegeben (pH 3.7). Die entstandene Lösung wird klarfiltriert und das Filtrat lyophilisiert.

Man erhält 2.7 g β-Cyclodextrin-Einschlussverbindung der (6R)-5,10-Methylentetrahydrofolsäure mit einem Gehalt von (6R)-5,10-Methylentetrahydrofolsäure von 15.5% (bestimmt mittels HPLC).

Stabilität Feststoff: Gehalt der (6R)-5,10-Methylentetrahydrofolsäure nach 158 Tg/- 25° C 100.0%.

### Beispiel 12

### β-Cyclodextrin-Einschlussverbindung des Sulfates der (6R)-5,10-Methylentetrahydrofolsäure

Durch die Verwendung von 540 mg Schwefelsäuresalz der (6R)-5,10-Methylentetrahydrofolsäure analog zu Beispiel 11 erhält man 2.65 g β-Cyclodextrin-Einschlussverbindung des Schwefelsäuresalzes der (6R)-5,10-Methylentetrahydrofolsäure mit einem Gehalt an (6R)-5,10-Methylentetrahydrofolsäure von 15.2% (bestimmt mittels HPLC).

Stabilität Feststoff: Gehalt an (6R)-5,10-Methylentetrahydrofolsäure-Sulfat nach 157 Tg/- 25° C 99.0%.

### Beispiel 13

### β-Cyclodextrin-Einschlussverbindung des Benzolsulfonates der (6R)-5,10-Methylentetrahydrofolsäure

Durch die Verwendung von 603 mg Benzolsulfonsäuresalz der (6R)-5,10-Methylentetrahydrofolsäure analog zu Beispiel 11 erhält man 2.94 g β-Cyclodextrin-Einschlussverbindung des Benzolsulfonsäuresalzes der (6R)-5,10-Methylentetrahydrofolsäure mit einem Gehalt an (6R)-5,10-Methylentetrahydrofolsäure von 13.1% (bestimmt mittels HPLC).

Stabilität Feststoff: Gehalt an (6R)-5,10-Methylentetrahydrofolsäure-Benzolsulfonat nach 72 Tg/- 25° C 100.0%.

### Beispiel 14

### β-Cyclodextrin-Einschlussverbindung des Calciumsalzes der (6R)-5,10-Methylentetrahydrofolsäure

Durch die Verwendung von 610 mg Calciumsalz der (6R)-5,10-Methylentetrahydrofolsäure analog zu Beispiel 11 erhält man 2.65 g β-Cyclodextrin-Einschlussverbindung des Calciumsalzes der (6R)-5,10-Methylentetrahydrofolsäure mit einem Gehalt an (6R)-5,10-Methylentetrahydrofolsäure von 15.6% (bestimmt mittels HPLC).

### Beispiel 15

### β-Cyclodextrin-Einschlussverbindung des Magnesiumsalzes der (6R)-5,10-Methylenfetrahydrofolsäure

Durch die Verwendung von 600 mg Magnesiumsalz der (6R)-5,10-Methylentetrahydrofolsäure analog zu Beispiel 11 erhält man 2.69 g β-Cyclodextrin-Einschlussverbindung des Magnesiumsalzes der (6R)-5,10-Methylentetrahydrnfolsäure mit einem Gehalt an (6R)-5,10-Methylentetrahydrofolsäure von 13.1% (bestimmt mittels HPLC).

Stabilität Feststoff: Gehalt an Magnesiumsalz der (6R)-5,10-Methylentetrahydrofolsäure nach 155 Tg/- 25° C 99.5%.

### Beispiel 16

### Dimethyl-β-Cyclodextrin-Einschlussverbindung des Natriumsalzes der (6R)-5,10-Methylentetrahydrofolsäure

26.2 g Dimethyl-β-Cyclodextrin werden in 100 ml Wasser gelöst. Bei Raumtemperatur werden unter Stickstoff 5.6 g (6R)-5,10-Methylentetrahydrofolsäure-Sulfat zugegeben. Dabei wird der pH der Lösung bei 7-9 gehalten (37 ml NaOH 1n). Die entstandene Lösung wird klarfiltriert und lyophilisiert.

Man erhält 32.6 g Dimethyl-β-Cyclodextrin-Einschlussverbindung des Natriumsalzes der (6R)-5,10-Methylentetrahydrofolsäure mit einem Gehalt an (6R)-5,10-Methylentetrahydrofolsäure von 14.3% (bestimmt mittels HPLC).

Stabilität Feststoff: Gehalt an Natriumsalz der (6R)-5,10-Methylentetrahydrofolsäure nach 56 Tg/- 25° C 100%; nach 56 Tg/+ 25° C 97.5%.

### Beispiel 17

### Dimethyl-β-Cyclodextrin-Einschlussverbindung des Schwefelsäuresalzes der (6R)-5,10-Methylentetrahydrofolsäure

2.62 g Dimethyl-β-Cyclodextrin werden in 10 ml Wasser gelöst. Bei Raumtemperatur werden unter Stickstoff 560 mg (6R)-5,10-Methylentetrahydrofolsäure-Sulfat und anschliessend 140 ml Wasser zugegeben. Die entstandene Lösung wird klarfiltriert und lyophilisiert.

Man erhält 3.29 g Dimethyl-β-Cyclodextrin-Einschlussverbindung des Schwefelsäuresalzes der (6R)-5,10-Methylentetrahydrofolsäure mit einem Gehalt an (6R)-5,10-Methylentetrahydrofolsäure von 13.6% (bestimmt mittels HPLC).

### Beispiel 18

### Hydroxypropyl-β-Cyclodextrin-Einschlussverbindungen der (6R)- oder (6S)-5,10-Methylentetrahydrofolsäure

Durch die Verwendung von Hydroxypropyl-β-Cyclodextrin analog zu den Beispielen 3-17 erhält man die Hydroxypropyl-β-Cyclodextrin-Einschlussverbindungen der (6R)-oder (6S)-5,10-Methylentetrahydrofolsäure und dessen Salzen.

### Beispiel 19

### Hydroxypropyl-γ-Cyclodextrin-Einschlussverbindungen der (6R) oder (6S)-5,10-Methylentetrahydrofolsäure

Durch die Verwendung von Hydroxypropyl-γ-Cyclodextrin analog zu den Beispielen 3-17 erhält man die Hydroxypropyl-γ-Cyclodextrin-Einschlussverbindungen der (6R)-oder (6S)-5,10-Methylentetrahydrofolsäure und deren Salzen.

### Beispiel 20

### Dimethyl-γ-Cyclodextrin-Einschlussverbindungen der (6R)- oder (6S)-5,10-Methylentetrahydrofolsäure

Durch die Verwendung von Dimethyl-γ-Cyclodextrin analog zu den Beispielen 3-17 erhält man die Dimethyl-γ-Cyclodextrin-Einschlussverbindung der (6R)- oder (6S)-5,10-Methylentetrahydrofolsäure und deren Salz.

## Patentansprüche

1. Stabile, wässrige Zusammensetzung beinhaltend:
i) (6R)-, (6S)- oder (6R,S)-5,10-Methylentetrahydrofolsäure oder ein Salz der (6R)-, (6S)- oder (6R,S)-5,10-Methylentetrahydrofolsäure und
ii a) α-, β- oder γ-Cyclodextrin oder ein Derivat von α-, β- oder γ-Cyclodextrin oder
ii b) ein Gemisch von α-, β- oder γ-Cyclodextrin oder ein Gemisch von Derivaten von α-, β- oder γ-Cyclodextrin oder ein Gemisch von α-, β- oder γ-Cyclodextrin mit einem Derivat von α-, β- oder γ-Cyclodextrin.

2. Zusammensetzung nach Anspruch 1, wobei (6R)-5,10-Methylentetrahydrofolsäure oder ein pharmazeutisch verträgliches Salz der (6R)-5,10-Methylentetrahydrofolsäure und β- oder γ-Cyclodextrin oder ein pharmazeutisch verträgliches Derivat von β- oder γ-Cyclodextrin eingesetzt wird.

3. Zusammensetzung nach Anspruch 1 oder 2, wobei als pharmazeutisch verträgliches Salz der 5,10-Methylentetrahydrofolsäure ein Alkali- oder Erdalkalisalz, vorzugsweise das Natrium-, Magnesium- oder Calciumsalz und als pharmazeutisch verträgliches Derivat von Cyclodextrin hydroxyalkyliertes oder alkyliertes β- oder γ-Cyclodextrin, vorzugsweise Hydroxypropyl-β-, Hydroxypropyl-γ- oder Dimethyl-β-Cyclodextrin eingesetzt wird.

4. α-, β- oder γ-Cyclodextrin-Einschlussverbindungen von (6R)- oder (6S)-5,10-Methylentetrahydrofolsäure oder deren Salzen.

5. Hydroxyalkyl-β- oder Hydroxyalkyl-γ-Cyclodextrin-Einschlussverbindung von (6R)- oder (6S)-5,10-Methylentetrahydrofolsäure oder deren Salzen.

6. Hydroxypropyl-β- oder Hydroxypropyl-γ-Cyclodextrin-Einschlussverbindungen von (6R)- oder (6S)-5,10-Methylentetrahydrofolsäure oder deren Salzen.

7. Alkyl-β- oder Alkyl-γ-Cyclodextrin-Einschlussverbindung von (6R)- oder (6S)-5,10-Methylentetrahydrofolsäure oder deren Salzen.

8. Dimethyl-β- oder Dimethyl-γ-Cyclodextrin-Einschlussverbindungen von (6R)-oder (6S)-5,10-Methylentetrahydrofolsäure oder deren Salzen.

9. Verfahren zur Stabilisierung von wässrigen Lösungen von (6R)-, (6S)- oder (6R,S)-5,10-Methylentetrahydrofolsäure und deren Salzen durch Zufügen von α-, β- oder γ-Cyclodextrin oder eines Derivates von α-, β- oder Cyclodextrin.

10. Verfahren zur Herstellung von stabilen Lösungen der (6R)-, (6S)- oder (6R,S)-5,10-Methylentetrahydrofolsäure oder deren Salzen, dadurch gekennzeichnet, dass man (6S)-, (6R)- oder (6R,S)-Tetrahydrofolsäure oder ein Salz der entsprechenden Tetrahydrofolsäure in Gegenwart von α-, β- oder γ-Cyclodextrin oder eines Derivats von α-, β- oder γ-Cyclodextrin mit Formaldehyd umsetzt, falls erwünscht umsalzt und falls erwünscht, die erhaltene 5,10-Methylentetrahydrofolsäure-Cyclodextrin-Einschlussverbindung isoliert.

11. Verwendung von (6R)-, (6S)- oder (6R,S)-5,10-Methylentetrahydrofolsäure oder deren Salzen als Einschlussverbindungen in α-, β- oder γ-Cyclodextrin oder deren Derivaten als Bestandteil oder als Ausgangsmaterial zur Herstellung von Arzneimitteln.

## Claims

1. Stable, aqueous composition comprising:
i) (6R)-, (6S)- or (6R,S)-5,10-methylenetetrahydrofolic acid or a salt of (6R)-, (6S)- or (6R,S)-5,10-methylenetetrahydrofolic acid and
iia) α-, β- or γ-cyclodextrin or a derivative of α-, β- or γ-cyclodextrin or
iib) a mixture of α-, β- or γ-cyclodextrin or a mixture of derivatives of α-, β- or γ-cyclodextrin or a mixture of α-, β- or γ-cyclodextrin with a derivative of α-, β-or γ-cyclodextrin.

2. Composition according to Claim 1, in which (6R)-5,10-methylenetetrahydrofolic acid or a pharmaceutically tolerable salt of (6R)-5,10-methylenetetrahydrofolic acid and β- or γ-cyclodextrin or a pharmaceutically tolerable derivative of β- or γ-cyclodextrin is used.

3. Composition according to Claim 1 or 2, in which the pharmaceutically tolerable salt of 5,10-methylenetetrahydrofolic acid employed is an alkali metal or alkaline earth metal salt, preferably the sodium, magnesium or calcium salt and the pharmaceutically tolerable derivative of cyclodextrin employed is hydroxyalkylated or alkylated β- or γ-cyclodextrin, preferably hydroxypropyl-β-, hydroxypropyl-γ- or dimethyl-β-cyclodextrin.

4. α-, β- or γ-cyclodextrin inclusion compounds of (6R)- or (6S)-5,10-methylenetetrahydrofolic acid or its salts.

5. Hydroxyalkyl-β- or hydroxyalkyl-γ-cyclodextrin inclusion compounds of (6R)- or (6S)-5,10-methylenetetrahydrofolic acid or its salts.

6. Hydroxypropyl-β- or hydroxypropyl-γ-cyclodextrin inclusion compounds of (6R)- or (6S)-5,10-methylenetetrahydrofolic acid or its salts.

7. Alkyl-β- or alkyl-γ-cyclodextrin inclusion compounds of (6R)- or (6S)-5,10-methylenetetrahydrofolic acid or its salts.

8. Dimethyl-β- or dimethyl-γ-cyclodextrin inclusion compounds of (6R)- or (6S)-5,10-methylenetetrahydrofolic acid or its salts.

9. Process for the stabilisation of aqueous solutions of (6R)-, (6S)- or (6R,S)-5,10-methylenetetrahydrofolic acid and its salts by adding α-, β- or γ-cyclodextrin or a derivative of α-, β- or γ-cyclodextrin.

10. Process for the preparation of stable solutions of (6R)-, (6S)- or (6R,S)-5,10-methylenetetrahydrofolic acid or its salts, characterised in that (6S)-, (6R)- or (6R,S)-tetrahydrofolic acid or a salt of the corresponding tetrahydrofolic acid is reacted with formaldehyde in the presence of α-, β- or γ-cyclodextrin or a derivative of α-, β- or γ-cyclodextrin, if desired converted into another salt and if desired the 5,10-methylenetetrahydrofolic acid-cyclodextrin inclusion compound obtained is isolated.

11. Use of (6R)-, (6S)- or (6R,S)-5,10-methylenetetrahydrofolic acid or its salts as inclusion compounds in α-, β- or γ-cyclodextrin or their derivatives as a constituent or as a starting material for the preparation of medicaments.

## Revendications

1. Composition aqueuse stable contenant :
i) de l'acide (6R)-, (6S)- ou (6R,S)-5,10-méthylène-tétrahydrofolique ou un sel de l'acide (6R)-, (6S)- ou (6R,S)-5,10-méthylène-tétrahydrofolique et
iia) de l'alpha-, de la bêta- ou de la gamma-cyclodextrine ou un dérivé de l'alpha-, de la bêta- ou de la gamma-cyclodextrine, ou bien
iib) un mélange d'alpha-, de bêta- ou de gamma-cyclodextrine ou un mélange de dérivés de l'alpha-, de bêta)- ou de gamma-cyclodextrine ou un mélange d'alpha-, de bêta- ou de gamma-cyclodextrine avec un dérivé de l'alpha-, de la bêta- ou de la gamma-cyclodextrine.

2. Composition selon revendication 1, pour laquelle on utilise l'acide (6R)-5,10-méthylène-tétrahydrofolique ou un sel de cet acide acceptable pour l'usage pharmaceutique et la bêta- ou la gamma-cyclodextrine ou un dérivé de bêta- ou gamma-cyclodextrine acceptable pour l'usage pharmaceutique.

3. Compositions selon revendication 1 ou 2, pour laquelle le sel de l'acide 5,10-méthylène-tétrahydrofolique convenant pour les usages pharmaceutiques est un sel alcalin ou alcalino-terreux, de préférence un sel de sodium, de magnésium ou de calcium, et le dérivé de cyclodextrine acceptable pour l'usage pharmaceutique est une bêta- ou gamma-cyclodextrine hydroxyalkylée ou alkylée, de préférence l'hydroxypropyl-bêta, l'hydroxypropyl-gamma- ou la diméthyl-bêta-cyclodextrine.

4. Composés d'inclusion de l'acide (6R)- ou (6S)-5,10-méthylène-tétrahydrofolique ou de leurs sels, et de l'alpha-, de la bêta- ou de la gamma-cyclodextrine.

5. Composés d'inclusion de l'acide (6R)- ou (6S)-5,10-méthylène-tétrahydrofolique ou de leurs sels et d'une hydroxyalkyl-bêta ou hydroxyalkyl-gamma-cyclodextrine.

6. Composés d'inclusion de l'acide (6R)- ou (6S)-5,10-méthylène-tétrahydrololique ou de leurs sels et de l'hydroxypropyl-bêta- ou de l'hydroxypropyl-gamma-cyclodextrine.

7. Composés d'inclusion de l'acide (6R)- ou (6S)-5,10-méthylène-tétrahydrofolique ou de leurs sels et d'une alkyl-bêta- ou alkyl-gamma-cyclodextrine.

8. Composés d'inclusion de l'acide (6R)- ou (6S)-5,10-méthylène-tétrahydroiolique ou de leurs sels et de la diméthyl-bêta- ou diméthyl-gamma-cyclodextrine.

9. Procédé pour stabiliser des solutions aqueuses d'acide (6R)-, (6S)- ou (6R,S)-5,10-méthylène-tétrahydrofolique ou de ses sels par addition d'alpha-de bêta- ou de gamma-cyclodextrine ou d'un dérivé d'alpha-, de bêta- ou de gamma-cyclodextrine.

10. Procédé pour préparer des solutions stables d'acide (6R)-, (6S)- ou (6R,S)-5,10-méthylène-tétrahydrofolique ou de ses sels, caractérisé en ce que l'on fait réagir l'acide (6S)-, (6R)- ou (6R,S)-tétrahydrofolique ou un sel d'un tel acide, en présence d'alpha-, de bêta- ou de gamma-cyclodextrine ou d'un dérivé d'alpha-, de bêta- ou de gamma-cyclodextrine, avec le formaldéhyde, si on le désire, on convertit en un sel et si on le désire, on isole le composé d'inclusion acide 5,10-méthylène-tétrahydrofolique-cyclodextrine ainsi obtenu.

11. Utilisation de l'acide (6R)-, (6S)- ou (6R,S)-5,10-méthylène-tétrahydrofolique ou un de ses sels à l'état de composés d'inclusion avec l'alpha-, la bêta-ou la gamma-cyclodextrine ou leurs dérivés, en tant que constituants de médicaments ou produits de départ de la préparation de médicaments.
